# NEUE EUROPÄISCHE PATENTSCHRIFT

(11) **EP 1 210 019 B2**
(45) Veröffentlichungstag und Bekanntmachung des Hinweises auf die Entscheidung über den Einspruch: **15.07.2009**
(45) Hinweis auf die Patenterteilung: 20.10.2004
(21) Anmeldenummer: 99939893.6
(22) Anmeldetag: 08.09.1999
(51) Int. Cl.: A61B 17/86

(54) **VERWENDUNG EINER KNOCHENSCHRAUBE**
USE OF A BONE SCREW
UTILISATION D'UNE VIS A OS

(43) Veröffentlichungstag der Anmeldung: 05.06.2002
(73) Patentinhaber: Synthes AG Chur, 7002 Chur (CH)
(72) Erfinder: REYNDERS, Piet, B-3000 Leuven (BE); BERGER, Roger, CH-3661 Guetendorf (CH)
(74) Vertreter: Lusuardi, Werther
(86) Internationale Anmeldenummer: PCT/CH1999/000419
(87) Internationale Veröffentlichungsnummer: WO 2001/017447

(56) Entgegenhaltungen:
- WO-A-00/28907
- WO-A-88/06023
- DE-A- 3 508 759
- US-A- 4 653 489
- US-A- 5 098 435
- PATENT ABSTRACTS OF JAPAN vol. 95, no. 5, 30. Juni 1995 (1995-06-30) & JP 07 051292 A (Y.KISHIGAMI), 28. Februar 1995 (1995-02-28)
- PATENT ABSTRACTS OF JAPAN vol. 95, no. 11, 26. Dezember 1995 (1995-12-26) & JP 07 222752 A (TERUMO), 22. August 1995 (1995-08-22)

## Beschreibung

Die Erfindung betrifft die Verwendung einer Knochenschraube gemäss Patentanspruch 1.
Aus der DE-A-3 508 759 ist eine Hüftschraube bekannt, welche eine durchgehende zentrale Bohrung aufweist, welche in die offene Schraubenspitze mündet und seitliche Perforationen zwischen den Gewindegangwänden aufweist. Mittels dieser Hüftschraube soll einerseits eine Verankerung des überdimensionierten und spitzen Gewindes im Knochen erreicht werden und gleichzeitig durch Einspritzen von Knochenzement in die zentrale Bohrung und Austritt in die seitlichen Perforationen eine verfestigung des geschwächten Knochenmaterials erreicht werden. Der Nachteil dieser Hüftschraube besteht darin, dass sie nach erfolgter Aushärtung des Zementes nicht mehr weiter axial nach vorne eingedreht werden kann, da der durch die offene Spitze ausgetretene, und ausgehärtete Zement dies nicht mehr zulässt.
Aus der JP-07051292-A sowie der JP-07222752-A sind ebenfalls zentral durchbohrte Knochenschrauben bekannt, welche allerdings über die gesamte Länge, d.h. von der Schraubenspitze bis zum Schraubenkopf Perforationen aufweisen. Der Nachteil dieser beiden Schrauben besteht darin, dass auch im proximalen Teil der Schrauben Knochenzement austritt, was klinisch unerwünscht ist. Hier will die Erfindung Abhilfe schaffen. Der Erfindung liegt die Aufgabe zugrunde, eine teilweise durchbohrte Knochenschraube zu verwenden, durch welche Knochenzement derart in den umliegenden Knochen einbringbar ist, dass sich ein künstliches, koaxiales Zementbett für das Gewinde der Knochenschraube ausbildet, welches jedoch nur lateral an die Schraube angrenzt, so dass diese auch nach erfolgter Erhärtung des Zementes weiter axial nach vorne eingedreht (nachgezogen) werden kann.

Die Erfindung löst die gestellte Aufgabe mit der Verwendung einer Knochenschraube, welche die Merkmale des Anspruchs 1 aufweist.

Damit ist der Vorteil erzielbar, dass die bereits fixierte Knochenschraube, wie dies insbesondere bei der Befestigung von Knochenplatten wünschbar ist, auch nach erfolgter Erhärtung des künstlichen Zementbettes nachgezogen werden kann, weil durch die verschlossene Schraubenspitze kein Knochenzement austreten kann, welcher die axiale Bewegung der Knochenschraube blockieren könnte.

Bei einer bevorzugten Ausführungsform der Erfindung ist der Flankenwinkel des Aussengewindes der Knochenschraube zweckmässigerweise grösser als 25° und liegt vorzugsweise im Bereich von 29° bis 61°.
Die Mittelachsen der Perforationen müssen, auf der Längsachse gemessen, einen Abstand von höchstens 0,5 L zur Schraubenspitze hin aufweisen.
Der Durchmesser des Aussengewindes sollte höchstens 7,5 mm betragen und vorzugsweise im Bereich von 3,0 bis 7,5 mm liegen.

Der Durchmesser der zentralen Bohrung sowie der Perforationen muss im Bereich von 0,9 bis 3,3 mm liegen. Die Anzahl der Perforationen liegt zwischen 3 bis 8 liegen; sie können vorzugsweise, relativ zur Längsachse, auf ein bis drei orthogonal dazu stehenden Ebenen angeordnet sein.

Die im Kopf der Knochenschraube mündende Öffnung der Bohrung kann als Mehrkant-Kavität ausgebildet sein, welche einen entsprechenden Mehrkant-Schraubenzieher aufnehmen kann. Die Öffnung kann als Steckverbindung für einen Adapter zur Kopplung mit einer Knochenzementspritze ausgebildet sein.

Das Verhältnis D/F zwischen Aussengewindedurchmesser D in mm und der Gesamtaustrittsfläche F der Perforationen in mm² liegt im Bereich von 0,1 bis 0,9 mm⁻¹ und das Verhältnis D/d zwischen Aussengewindedurchmesser D und dem Durchmesser d der Bohrung muss im Bereich von 2,0 bis 2,9 liegen.

Die Perforationen reichen zweckmässigerweise bis in den Bereich der Gewindegangwände.

Die erfindungsgemässe Verwendung der Knochenschraube kann in bekannter Weise zur Fixation von Knochenplatten mit mehreren durchgehenden Plattenbohrungen dienen.

Die Erfindung und Weiterbildungen der Erfindung werden im folgenden anhand der teilweise schematischen Darstellung eines Ausführungsbeispiels noch näher erläutert.

Es zeigt:

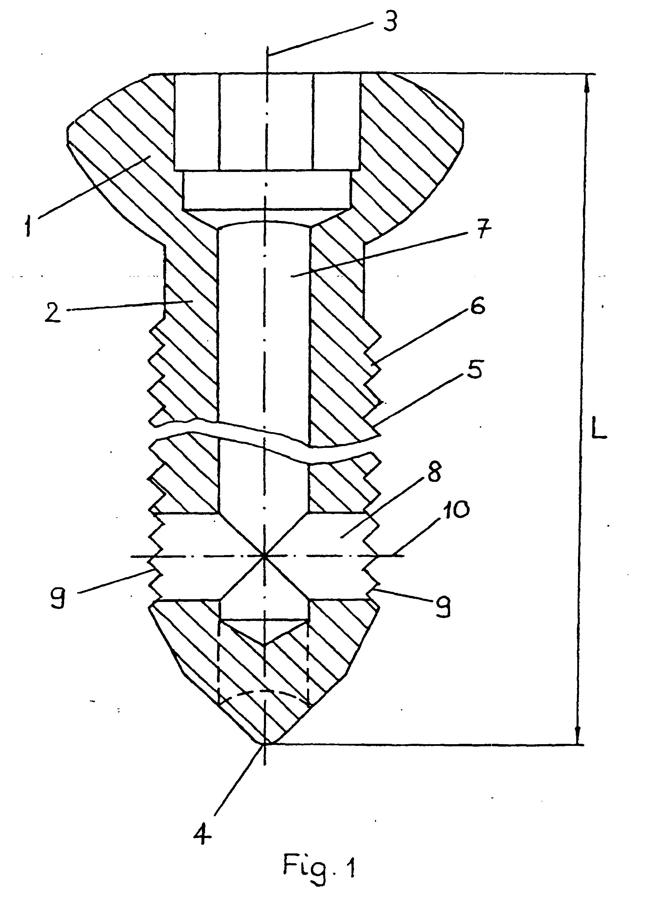

Fig. 1 einen Längsschnitt durch eine Knochenschraube zur erfindungsgemässen Verwendung.

Die Knochenschraube mit der zentralen Längsachse 3 und der Gesamtlänge L weist einen Kopf 1 auf, an welchen ein Schaft 2 mit einer Schraubenspitze 4 anschliesst. Die Mantelfläche 5 des Schaftes 2 weist ein Aussengewinde 6 auf. Koaxial zur Längsachse 3 verläuft eine, gegen den Kopf 1 hin offene Bohrung 7, welche über einen, quer zur Längsachse 3 verlaufenden Kanal 8 mit der Mantelfläche 5 des Schaftes 2 verbunden ist und in Form von Perforation 9 durch die Mantelfläche 5 nach aussen mündet. Die Bohrung 7 ist zur Schraubenspitze 4 hin verschlossen.
Die Mittelachsen 10 der Perforationen 9 weisen, auf der Längsachse 3 gemessen, einen Abstand von ca. 0,28 L zur Schraubenspitze 4 hin auf.
Die im Kopf 1 der Knochenschraube mündende Öffnung der Bohrung 7 ist als Sechskant-Kavität ausgebildet, welche einen entsprechenden Schraubenzieher aufnehmen kann..

## Patentansprüche

1. Verwendung einer Knochenschraube zum Einbringen von Knochenzement in den umliegenden Knochen mit einem Kopf (1), einem Schaft (2), einer zentralen Längsachse (3), einer Schraubenspitze (4), einer Mantelfläche (5), einem Aussengewinde (6) und einer Gesamtlänge L, wobei eine koaxial zur Längsachse (3) verlaufende, gegen den Kopf (1) hin offene Bohrung (7) vorgesehen ist, welche über mindestens einen, quer zur Längsachse (3) verlaufenden Kanal (8) mit der Mantelfläche (5) verbunden ist und in Form von mindestens einer Perforation (9) durch die Mantelfläche (5) nach aussen mündet, wobei die Bohrung (7) zur Schraubenspitze (4) hin verschlossen ist, und die Mittelachsen (10) der Perforationen (9), auf der Längsachse (3) gemessen, einen Abstand von höchstens 0,5 L zur Schraubenspitze (4) ausweisen; wobei
A) das Verhältnis D/F zwischen Aussengewindedurchmesser D in mm und der Gesamtaustrittsfläche F der Perforationen (9) in mm² im Bereich von 0,1 bis 0,9 mm⁻¹ liegt;
B) der Durchmesser der Perforationen (9) im Bereich von 0,9 bis 3,3 mm liegt;
C) das Verhältnis D/d zwischen Aussengewindedurchmesser D und dem Durchmesser d der Bohrung (7) im Bereich von 2,0 bis 2,9 liegt; und
D) die Anzahl der Perforationen (9) im Bereich von 3 bis 8 liegt.

2. Verwendung nach Anspruch 1, **dadurch gekennzeichnet, dass** der Flankenwinkel des Aussengewinde (6) grösser als 25° ist, und vorzugsweise im Bereich von 29° bis 61 ° liegt.

3. Verwendung nach Anspruch, 1 oder 2, **dadurch gekennzeichnet, dass** der Durchmesser des Aussengewindes (6) höchstens 7,5 mm beträgt.

4. Verwendung nach einem der Ansprüche 1 bis 3, **dadurch gekennzeichnet, dass** die Perforationen (9), relativ zur Längsachse (3), auf ein bis drei orthogonal dazu stehenden Ebenen angeordnet sind.

5. Verwendung nach einem der Ansprüche 1 bis 4, **dadurch gekennzeichnet, dass** die im Kopf (1) mündende Öffnung der Bohrung (7) als Steckverbindung für einen Adapter zur Kopplung mit einer Knochenzementspritze ausgebildet ist.

6. Verwendung nach einem der Ansprüche 1 bis 5, **dadurch gekennzeichnet, dass** die Perforationen (9) in den Bereich der Gewindegangwände reichen.

7. Verwendung nach Anspruch 5, mit einem Adapter zur Kopplung mit einer Knochenzementspritze.

## Claims

1. Use of a bone screw for introducing bone cement in the surrounding bone with a head (1), a shank (2), a central longitudinal axis (3), a screw tip (4), a jacket surface (5), an external thread (6), and a total length (L), being provided with a bore (7) which is coaxial with the longitudinal axis (3) and open towards the head (1) and which is connected to the jacket surface (5) via at least one channel (8) extending transversely to the longitudinal axis (3) and opens towards the outside in the form of at least one perforation (9) through the jacket surface (5), whereby the bore (7) is closed towards the screw tip (4), and the central axes (10) of the perforations (9), measured on the longitudinal axis (3), are at a distance of at most 0.5 L from the screw tip (4); wherein
A) the ratio D:F between external thread diameter D in mm and the total exit area F of the perforations (9) in mm² is in the range of 0.1 to 0.9 mm⁻¹;
B) the diameter of the perforations (9) lies in the range of 0.9 to 3.3 mm;
C) the ratio D:d between external thread diameter D and the diameter d of the bore (7) lies in the range of 2.0 to 2.9; and
D) the number of perforations (9) lies in the range of 3 to 8.

2. Use according to Claim 1, **characterized in that** the flank angle of the external thread (6) is greater than 25°, and preferably lies in the range of 29° to 61°.

3. Use according to one of Claims 1 to 2, **characterized in that** the diameter of the external thread (6) is at most 7.5 mm.

4. Use according to one of Claims 1 to 3, **characterized in that** the perforations (9), relative to the longitudinal axis (3), are arranged on one to three planes oriented at right angles thereto.

5. Use according to one of Claims 1 to 4, **characterized in that** the opening of the bore (7) opening out in the head (1) is designed as a socket connection for an adapter for coupling to a bone cement syringe.

6. Use according to one of Claims 1 to 5, **characterized in that** the perforations (9) reach into the area of the thread walls.

7. Use according to Claim 5 with an adapter for coupling to a bone cement syringe.

## Revendications

1. Utilisation d'une vis pour ostéosynthèse pour introduire du ciment à os dans l'os environnante avec une tête (1), une tige (2), un axe longitudinal central (3), une pointe de vis (4), une surface de gaine (5), un filetage mâle (6) et une longueur totale L, un alésage (7) coaxial par rapport à l'axe longitudinal (3) et ouvert en direction de la tête (1) étant prévu, lequel est relié à la surface de gaine (5) par au moins un conduit (8) transversal à l'axe longitudinal (3) et débouche vers l'extérieur sous forme d'au moins une perforation (9) à travers la surface de gaine (5), l'alésage (7) étant obturé en direction de la pointe de la vis (4) et la distance maximale, mesurée sur l'axe longitudinal (3), entre les axes médians (10) des perforations(9) et la pointe de la vis (4) étant de 0,5 L; où
A) le rapport D/F entre le diamètre externe du filetage mâle D en mm et la surface totale de sortie F des perforations (9) en mm² est compris entre 0,1 et 0,9 mm⁻¹ ;
B) le diamètre des perforations (9) est compris entre 0,9 et 3,3 mm ;
C) le rapport D/d entre le diamètre D du filetage mâle et le diamètre d de l'alésage (7) est compris entre 2,0 et 2,9 ; et
D) le nombre de perforations (9) est compris entre 3 et 8.

2. Utilisation selon la revendication 1, **caractérisée en ce que** l'angle du filet du filetage mâle (6) est supérieur à 25°, et de préférence compris entre 29° et 61 °.

3. Utilisation selon une des revendications 1 à 2, **caractérisée en ce que** le diamètre du filetage mâle (6) n'excède pas 7,5 mm.

4. Utilisation selon une des revendications 1 à 3, **caractérisée en ce que** les perforations (9) sont disposées sur un à trois plans orthogonaux par rapport à l'axe longitudinal (3).

5. Utilisation selon une des revendications 1 à 4, **caractérisée en ce que** l'orifice de l'alésage (7) qui débouche dans la tête (1) est conçu comme récepteur pour un adaptateur de couplage avec un pistolet pour ciment à os.

6. Utilisation selon une des revendications 1 à 5, **caractérisée en ce que** les perforations (9) atteignent la zone des parois du pas de vis.

7. Utilisation selon la revendication 5, avec un adaptateur de couplage avec un pistolet pour ciment à os.
